(19) Europäisches Patentamt
European Patent Office
Office européen des brevets

(11) Publication number: **0 176 330 B1**

(12) # EUROPEAN PATENT SPECIFICATION

(45) Date of publication of patent specification: **18.07.90**

(51) Int. Cl.[5]: **A 61 K 7/50**, C 11 D 10/04

(21) Application number: **85306730.4**

(22) Date of filing: **23.09.85**

(54) **Cleaning compositions with skin protection agents.**

(30) Priority: **25.09.84 US 654291**

(43) Date of publication of application:
**02.04.86 Bulletin 86/14**

(45) Publication of the grant of the patent:
**18.07.90 Bulletin 90/29**

(84) Designated Contracting States:
**AT BE CH DE FR GB IT LI NL SE**

(56) References cited:
**CH-A- 479 695**
**DE-A-2 402 223**
**FR-A-1 548 302**
**FR-A-1 580 491**
**LU-A- 55 496**
**US-A-3 043 779**
**US-A-3 607 766**

**The file contains technical information submitted after the application was filed and not included in this specification**

(73) Proprietor: **UNILEVER PLC**
**Unilever House Blackfriars P.O. Box 68**
**London EC4P 4BQ (GB)**
(84) **GB**

(73) Proprietor: **UNILEVER NV**
**Burgemeester s'Jacobplein 1 P.O. Box 760**
**NL-3000 DK Rotterdam (NL)**
(84) **BE CH DE FR IT LI NL SE**

(72) Inventor: **Caswell, Michael Lynn**
**219 Oak Street**
**Ridgewood New Jersey 07450 (US)**
Inventor: **Corr, James Joseph**
**15 Euclid Avenue**
**Huntington New York (US)**
Inventor: **Dobrovolny, Mark Stephen**
**1535 Raspberry Court**
**Edison New Jersey (US)**
Inventor: **Lander, Lynn Howard**
**268 Brook Street**
**Harrington Park New Jersey (US)**
Inventor: **Narath, William Robert**
**10 Alba Place**
**Parsippany New Jersey (US)**
Inventor: **Thetler, Richard Frederic**
**50 South Colonial Drive**
**Harrington Park New Jersey (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 176 330 B1**

(74) Representative: **Thomas, Susan Margaret et al**
**UNILEVER PLC Patents Division P.O. Box 68**
**Unilever House**
**London EC4P 4BQ (GB)**

**Description**

The present invention relates to cleaning compositions containing soap and mildness improving component.

Although a soap is efficient at cleaning, it requires formulation to overcome many physical defects. Additives have been discovered which improve soap's lather, fragrance, visual appeal and other aesthetic properties.

More recently, attention has been drawn to the harshness problem of soap toward skin. Eighteen well-known toilet soaps were evaluated by Frosch & Kligman, "J. Amer. Acad. Derm." pp 35 (1979). Great differences were noted in their effect upon skin. Most had an appreciable irritancy. The study revealed that substantial replacement of soap with an alternative detergent such as acyl fatty isethionate would provide a more skin compatible system. Unfortunately, this alternative is expensive. Cheaper solutions to the harshness problem would be desirable.

Reports of blending soap with acyl fatty isethionates, presumably to lower costs, have been numerous. US Patent 2 894 912 (Geitz) extols the virtues of toilet bars containing from 30 to 70% acyl fatty isethionate and 2.5 to 25% soap. In US Patent 4 260 507 (Barrett), a composition with major amounts of soap, 60—97%, was combined with minor amounts, 3—40%, acyl fatty isethionate. These toilet bars were claimed to have exceptional lathering properties.

Acyl fatty isethionate can be prepared by direct interesterification of $C_{12}$—$C_{25}$ fatty acid with the alkali metal salt of isethionic acid (known also as hydroxyethane sulfonic acid). Unesterified alkali metal isethionate itself has been reported as a toilet bar processing aid in US Patent 4 180 470 (Tokosh et al) in a toilet bar having an acyl isethionate as the predominate surface active detergent. US Patent 3 043 779 describes a detergent composition in the form of a soap bar or cake specially formulated to reduce the tendency to become soft and sticky during use. The formulation disclosed requires in addition to the soap the presence of water soluble alkali metal fatty-acyl-aminoethane sulphonate wherein the fatty-acyl radical has from about 8 to about 18 carbon atoms and a small proportion of a water-soluble salt of an organic monocarboxylic or monosulphonic acid having from 1 to 8 carbon atoms. An example of the latter is given as sodium isethionate.

According to a first aspect of the present invention there is provided a cleaning composition comprising:

(a) a fatty acid soap in an amount of from 30 to 98 wt%;

(b) a mildness improving salt of following structure:

$$HO{-}CHRCH_2{-}SO_3M$$

where R is hydrogen or a $C_1$—$C_7$ alkyl or alkenyl radical; M is a cation selected from among alkali metal, alkaline earth metal, ammonium, alkyl ammonium and mono-, di- or tri-alkanolammonium ions, in an amount of from 0.5 to 50 wt%; and wherein the ratio of soap to mildness improving salt ranges from 1:2 to 200:1.

(c) $C_8$—$C_{18}$ free fatty acid in an amount of from 1 to 30 wt%; the composition containing no water soluble alkali metal fatty-acylaminomethane sulphonate wherein the fatty-acyl radical has from 8 to 18 carbon atoms.

The present invention can thus provide a low cost cleaning composition containing a substantial amount of soap which composition can be milder to the skin than pure soap. The compositions presently described and claimed contain no water-soluble alkali metal fatty-acylaminomethane sulphonate wherein the fatty-acyl radical has from about 8 to about 18 carbon atoms.

DETAILED DESCRIPTION OF THE INVENTION

By use of the present composition, it has been found that non-acylated isethionate salts when incorporated into soap formulations can prevent skin damage, which can be a problem normally associated with unmodified soap. Unlike acyl fatty isethionates, their non-acylated progenitors can impart skin mildness at a concentration level significantly below that achieved through the acylated esters.

The term "soap" is used herein in its popular sense, i.e. the alkali metal or alkanol ammonium salts of alphatic alkane- or alkene monocarboxylic acids. Sodium, potassium, mono-, di- and tri-ethanol ammonium cations, or combinations thereof, are suitable for use in the present composition. In general, sodium soaps are preferred. From 1% to 25% of the soap may however suitably be potassium soaps. The soaps employed are preferably the well known alkali metal salts of natural or synthetic alphatic (alkanoic or alkenoic) acids having 12 to 20 carbon atoms, preferably 12 to 18 carbon atoms. They may be described as alkali metal carboxylates of acyclic hydrocarbons having 12 to 20 carbon atoms.

Soaps having the fatty acid distribution of coconut oil may for example provide the lower end of the broad molecular weight range. Those soaps having the fatty acid distribution of peanut or rapeseed oil, or their hydrogenated derivatives, may for example provide the upper end of the broad molecular weight range.

It is preferred to use soaps having the fatty acid distribution of coconut oil or tallow, or mixtures thereof, since these can be among the more readily available fats. The proportion of fatty acids having at

least 12 carbon atoms in coconut oil soap is about 85%. This proportion will be greater when mixtures of coconut oil and fats such as tallow, palm oil, or non-tropical nut oils or fats are used, wherein the principle chain lengths are $C_{16}$ and higher. Preferred soap for use in the present compositions has at least about 85% fatty acids having 12—18 carbon atoms.

Coconut oil employed for the soap may be substituted in whole or in part by other "high-lauric" oils, that is, oils or fats wherein at least 50% of the total fatty acids are composed of lauric or myristic acids and mixtures thereof. These oils are generally exemplified by the tropical nut oils of the coconut oil class. For instance, they include: palm kernel oil, babassu oil, ouricuri oil, tucum oil, cohune oil, murumuru oil, jaboty kernel oil, khakan kernel oil, dika nut oil, and ucuhuba butter.

A preferred soap is a mixture of 15% to 20% coconut oil and 80% to 85% tallow. These mixtures contain 95% fatty acids having 12 to 18 carbon atoms. The soap may alternatively be prepared from coconut oil, in which case the fatty acid content is about 85% of $C_{12}$—$C_{18}$ chain length.

The soaps may contain unsaturation in accordance with commercially acceptable standards. Excessive unsaturation is normally avoided.

Soaps may be made by the classic kettle boiling process or modern continuous soap manufacturing process wherein natural fats and oils such as tallow or coconut oil or their equivalents are saponified with an alkali metal hydroxide using procedures well known to those skilled in the art. Alternatively, the soaps may be made by neutralising fatty acids, such as lauric ($C_{12}$), myristic ($C_{14}$), palmitic ($C_{16}$), or stearic ($C_{18}$) acids with an alkali metal hydroxide or carbonate.

Total soap content of the present compositions must be from 30% to 98%. More preferably, the concentration of soap ranges from 50% to 70%.

Also present in an amount of from 1 to 30 wt% is a free fatty acid containing from 8 to 18 carbon atoms; preferably $C_{10}$—$C_{16}$ fatty acid is present in an amount up to 25 wt%. To improve skin feel and creaminess, stearic acid may for example be incorporated into the composition.

Cleaning compositions encompassed by the present invention may for example be in liquid or toilet bar form.

Skin mildness improvers hereby disclosed are salts of isethionate. Effective salt cations are selected from alkali metal, alkaline earth metal, ammonium, alkyl ammonium and mono-, di- or tri-alkanolammonium ions. Specifically preferred cations include sodium, potassium, lithium, calcium, magnesium, ammonium, triethylammonium, monoethanolammonium, diethanolammonium or triethanolammonium ions.

Particularly preferred as a mildness improver is simple, unsubstituted sodium isethionate of the general formula wherein R is hydrogen.

The mildness improver is preferably present from 0.5% to 50%. More preferably, the mildness improver is present from 1% to 25%, even more preferably from 2% to 15%, even more preferably from 6 to 15% by weight of the total composition.

Detergents other than soap may additionally be present in the composition. Their presence is, however, preferably no greater than the amount of soap present. The detergents may be chosen from the alkali metal, magnesium or ammonium salts selected from

$C_{12}$—$C_{16}$ hydroxyalkane sulfonates
$C_8$—$C_{18}$ acyl isethionates
$C_8$—$C_{18}$ N-acyl taurinates
$C_{12}$—$C_{18}$ alkyl sulfates
$C_{12}$—$C_{18}$ alkyl ether sulfates
$C_{12}$—$C_{16}$ alkyl phosphonates and phosphates
$C_{12}$—$C_{16}$ mono-alkyl succinates and maleates
$C_6$—$C_{14}$ dialkylsulfosuccinates
$C_{16}$—$C_{20}$ alkane disulfonates, and
$C_8$—$C_{18}$ alkene sulfonates.

Particularly preferred are the $C_8$—$C_{18}$ acyl isethionates, especially sodium $C_8$—$C_{18}$ acyl isethionates. These esters are prepared by reaction between alkali metal isethionate with mixed aliphatic fatty acids having from 6 to 18 carbon atoms and an iodine value of less than 20. At least 75% of the mixed fatty acids have from 12 to 18 carbon atoms and up to 25% have from 6 to 10 carbon atoms.

Acyl isethionates, when present, will generally range from 5%, preferably 10% to 40% by weight of the total composition. Preferably, this component is present from 15% to 30%.

Other performance chemicals may be included in the present compositions. For instance, from 2 to 10 wt% of a suds-boosting detergent salt may be incorporated. Illustrative of this type of additive are salts selected from the group consisting of alkali metal and organic amine higher aliphatic fatty alcohol sulfates, alkyl aryl sulfonates, and the higher aliphatic fatty acid taurinates.

Adjunct materials including germicides, perfumes, colourants, pigments such as titanium dioxide and water may also be present.

Compositions embodying the present invention can be prepared by for example adding the mildness improving salt and any other ingredients required to neat soap at 100 to 110°C in a high shear mixer. Subsequent process steps can for example follow conventional soap tablet making practice i.e. reducing moisture content, cooling, milling, plodding and stamping to form bars.

According to another aspect of the present invention there is provided a method for protecting skin against chemical damage comprising topically applying to the skin in an effective amount to prevent skin damage, a protective salt of the following structure:

$$HO — CHRCH_2 — SO_3M$$

where R is hydrogen or a $C_1$—$C_7$ alkyl or alkanyl radical; where M is a cation selected from either alkali metal, alkaline earth metal, ammonium, alkyl ammonium and mono-, di- or tri-alkanolammonium ions.

SKIN MILDNESS TESTS

Frosch-Kligman Soap Chamber Test

The Frosch-Kligman Soap Chamber Test is designed to evaluate the mildness of surfactant compositions on individuals with hypersensitive skin. An individual is deemed hypersensitive if, after occlusion with a 0.75% sodium lauryl sulfate patch for six hours, the treated site appears confluently red twenty-four hours after patch is applied. Approximately 30% of those screened for this study were found to have hypersensitive skin. Twenty-nine hyper-reactive qualified panellists participated in the experiments of Example 1.

Hill Top Chambers (Trade Mark) (25 mm diameter) were affixed to Dermicel (Trade Mark) Hypoallergenic cloth tape (Johnson & Johnson) to create an occluded patch. A Gilson (Trade Mark) micropipette was used to deliver 0.20 ml of solution to each of the respective chambers. Each of the panellists was assigned one of 31 randomised patched sequences.

Following removal of the patch on Day 5, each patched site was assessed by three trained judges 3 hours after patch removal. Three categories were evaluated according to the following (assessment) scales:

| Erythema | Scaling | Fissuring |
|---|---|---|
| 0 = no erythema | 0 = no scaling | 0 = no fissuring |
| 1 = slight redness, diffuse | 1 = fine | 1 = fine cracks |
| 2 = moderate, | 2 = moderate | 2 = single or uniform multiple broad fissures |
| 3 = intense redness | 3 = severe with large flakes | 3 = wide cracks with haemorrhage or exudation |

A preference rating for each site was also made. Panellists were assessed again on Day 8. These data were statistically analysed using a non-parametric Friedman's Test and Nemenyi's Procedure.

Guinea Pig Immersion Tests

The Guinea Pig Immersion Test has also been used as a predictive model for assessing skin irritation resulting from detergent insult.

Adult male albino Hartley guinea pigs served as the animal panellists. They were fed standard guinea pig chow and tap water *ad libitum* except during treatment periods. Prior to testing, the animals were observed for signs of skin defects and general disease. The animals were then acclimated in the facility for five days before start of the immersion treatments. During the treatment period, skin temperature and animal weight was monitored. An evaporimeter was used to measure transepidermal water loss. Skin thickness and surface pH were also measured. Laboratory conditions were maintained at 72° ± 2°F (22°C ± 1°C) and approximately 50% room humidity. Lighting was synchronised to 12 hours light followed by 12 hours darkness. Body weights were taken daily. Each animal was observed daily for sickness, and assesed for skin abnormalities. On the first and each subsequent day of experimentation, the abdominal surface of the guinea pig was closely clipped. Following assessments, the guinea pig was placed in a perforated canister with a "lock on" lid. The caged guinea pig was then placed in a 2-litre clear plastic Nalgene beaker containing circa 1.4 litres of pre-heated (38—40°C) immersion solution. This volume allowed immersion up to the thoracic axilla of each animal. Guinea pigs were immersed for 30 minutes with the immersion tank water held at 40°C. Immediately thereafter, each animal was removed from the immersion beaker, transferred to a 10 litre bucket of distilled water (40°C) and vigorously rinsed. The animal was then removed from its container, partially dried with paper towels and placed for thirty minutes in an infrared heated (90°F/32°C) incubator. After completion of the heated incubation period, the animal was returned to its cage. Three hours after initiating the first immersion, a second identical immersion procedure was executed. Tests continued for a period of 10—12 days.

After each immersion, the skin was rated for erythema, flaking and roughness response. Relative response scores ratings are outlined below:

| Erythema | Flaking | Roughness |
|---|---|---|
| 0 = No effect | 0 = No response | 0 = Smooth, normal response |
| 1 = Slight | 1 = Slight response | 1 = Slight response |
| 2 = Moderate | 2 = Moderate scaling | 2 = Moderate response |
| 3 = Severe | 3 = Moderate scaling with some sloughing of epidermis | 3 = Definite response |
| 4 = Severe with haemorrhage | 4 = Severe scaling sloughing of epidermis, marked cracking | 4 = Definite roughness with cracking |
| 5 = Necrosis | 5 = Sloughing of large areas of epidermis, deep cracking with possible haemorrhage | 5 = Severe roughness with deep cracking and oozing |

Within each category of skin damage, responses were averaged for the full 10—12 day period. Examples in this specification record the relative response scores averaged from the average of each animal.

Flex Wash Test

The Flex Wash procedure consists of three daily 60 second washes if the antecubital fossa (flex area of elbow). This method is designed to produce erythema quickly. Erythemal response varies only slightly with temperature and humidity fluctuations, unlike the Frosch-Kligman Test, making the protocol suitable for year round testing.

Approximately 20 male panellists were used as the test population. Panellist flex areas must be free of any skin condition (eczema, dryness, irritation, cuts or abrasions). Anyone taking antihistamines, antiinflammatory drugs (more than 8 per week) or topical, oral or injectable cortisone on a regular basis was excluded from the study. The panel was divided into two subgroups which are balanced for left handedness. Group I was assigned composition "A" for the left flex and "B" for the right flex. Group II reversed the order.

Following an evaluation, the panellist was instructed to moisten the left flex area. Sponge and test compositions when formulated as toilet bars were dampened with tap water (100 ppm calcium/magnesium ions). The sponge was then stroked over the test bar 10 times by the evaluator. The "dosed" sponge was placed in the panellist's right hand. Panellist then washed his left flex area for exactly 60 seconds (approximately 120 strokes). Thereupon, the flex was rinsed and patted dry. This washing procedure was repeated on right arm with the appropriate composition. Washing by this procedure was repeated 3 times daily for 5 consecutive days or a total of 15 washes. Treatment times were scheduled 1.5 hours apart. Each test site was evaluated immediately prior to washing and 4 hours after the third daily wash.

One trained assessor evaluated test sites prior to each wash and 4 hours after third wash of each day for a total of 20 evaluations. The grading scale was as follows:

0 = no erythema
0.5 = barely perceptible erythema
1 = mild spotty erythema/no oedema
1.5 = mild/moderate erythema/with or without oedema
2 = moderate confluent erythema/with or without oedema or vesiculation
2.5 = moderate/deep erythema/oedema/vesiculation
3 = deep erythema/oedema/vesiculation/weeping

Each site was treated in the prescribed method until a grading of "2" or greater was attained or 15 washings had been completed. When a score of "2" or greater was attained the treatment was discontinued on that flex. The final score was then carried through for all remaining evaluations. The remaining flex was washed until either a grading of at least "2" or 15 treatments were attained, whichever was first. In the Examples, the final grading is the sum total of grade scores for 20 assessments per panellist averaged over the scores from all panellists. Thus, theoretically the average score could range from 0 to 60; the lower value indicating absolutely no skin irritation while the latter being severe. In practice, scores generally ranged from about 15 to 30.

The following examples more fully illustrate embodiments of the present invention. All parts, percentages and proportions referred to herein and in the appended claims are by weight of the total composition unless otherwise stated.

Example 1

Sodium isethionate was evaluated herein for its mildness properties in combating the harsh effects of

soap in the skin. The formulations were tested; one was a control composition of 82/18 soap (tallow: coconut), the other additionally containing 15% sodium isethionate. Eight percent aqueous solutions were prepared by warming the compositions to 40°C and preparing blind labels for each sample. A Frosch-Kligman Soap Chamber Test was conducted according to the procedure outlined, *vide supra*. Results of these tests are listed as Mean Assessment and Preference Scores in Tables I and II, respectively.

TABLE I

Frosch-Kligman Test Results with Aqueous Soap Solutions and Sodium Isethionate

| | Mean Assessment Score | | | | | |
|---|---|---|---|---|---|---|
| | Erythema | | Scaling | | Fissuring | |
| Sodium Isethionate | 0% | 15% | 0% | 15% | 0% | 15% |
| Day 5 | 0.88 | 0.58 | 0.94 | 0.64 | 0.61 | 0.41 |
| Day 8 | 0.23 | 0.17 | 0.56 | 0.36 | 0.14 | 0.06 |

TABLE II

Frosch-Kligman Test Results With Aqueous Soap Solutions and Sodium Isethionate

| | Mean Preference Score | | | | | |
|---|---|---|---|---|---|---|
| | Erythema | | Scaling | | Fissuring | |
| Sodium Isethionate | 0% | 15% | 0% | 15% | 0% | 15% |
| Day 5 | 3.79 | 2.47 | 4.12 | 2.95 | 3.91 | 2.29 |
| Day 8 | 3.77 | 3.00 | 4.23 | 2.68 | 4.03 | 2.18 |

Values shown in Tables I and II were statistically significant at the 90% confidence level except for the fissuring assessment score. These tests were, because of necessity, performed during summer months when skin response to the Frosch-Kligman procedure is poorest. Response during the winter months usually provide scores approximately 2 to 7 times greater. Accordingly, the differences illustrated by Tables I and II between sodium isethionate containing soap compositions and those without are minimum values. They would be expected to be much greater under a more taxing winter environment.

Erythema assessment scores from Table I demonstrate that an 82/18 soap with 15% isethionate is significantly milder on Day 5 than a control without isethionate. Scaling was also noticeably less using the 15% isethionate soap composition. Although fissuring assessment scores were not statistically different, fissuring preference scores did differentiate 15% isethionate as providing a statistically measurable improvement in this skin condition. Levels of 5 and 10% sodium isethionate were also evaluated but not recorded in the Tables. Increased improvement in erythema, scaling and fissuring for both assessment and preference scores were noted as the level of isethionate rose.

Example 2

Illustrations of the personal washing compositions in the form of toilet bars are provided below.

The compositions also illustrate the presence of sodium acyl isethionate as a further detergent component, albeit minor, in the present soap/sodium isethionate compositions.

Compositions 1 and 2 were made by adding the minor ingredients listed to neat soap at about 100 to 110°C in a high shear mixer. The moisture content was reduced by evaporation to the required value. The mixture was then cooled on a chilled roller, milled, plodded into billets and stamped to form bars.

TABLE III

Toilet Bar Formulations Containing Soap/Acyl Isethionate/Sodium Isethionate

| Components | Sample 1 (wt%) | Sample 2 (wt%) | Sample 3 (wt%) | Sample 4 (wt%) |
|---|---|---|---|---|
| Sodium soap (82/18) | 46.53 | 54.27 | -- | -- |
| Sodium soap (60/40) | -- | -- | 62.9 | -- |
| Potassium soap (60/40) | -- | -- | 15.7 | -- |
| Sodium Soap | -- | -- | -- | 79.5 |
| Sodium acyl isethionate (45/55) | 19.94 | 23.26 | -- | -- |
| Sodium isethionate | 10.00 | 2.14 | -- | -- |
| Coconut fatty acid | 1.21 | 1.41 | -- | -- |
| Tallow/Coconut Fatty Acid (80/20) | -- | -- | -- | 7.4 |
| Stearic Acid | 6.40 | 7.47 | -- | -- |
| Sodium chloride | 0.40 | 0.46 | 1.0 | 1.4 |
| Water | 14.00 | 9.00 | 18.7 | 9.2 |
| Miscellaneous (Perfume, colourants, preservatives) | 1.52 | 1.99 | 1.0 | 2.1 |

Sample compositions 3 and 4 are well known toilet soaps; they served as control bars. Ratios associated with the sodium and potassium soaps refer to their relative content of tallow to coconut fatty acid residues.

Example 3

Frosch-Kligman Soap Chamber Tests were conducted on Samples 1—4 of Example 2. The procedure was as outlined previously with the exception that 42 qualified panellists participated in the experiments and each was assigned one of 20 randomised patch sequences. Also, unlike the solutions of Example 1, toilet bars were used in this Example and grated into 40°C water to provide 8% soap solutions (w/v). Results of the Chamber tests are recorded in Table IV, V and VI.

TABLE IV

Mean Erythema Scoring

Preference

| Sample: | 1 | 2 | 3 | 4 |
|---|---|---|---|---|
| Day 5 | 2.202 | 2.786 | 3.012 | 4.678 |
| Day 8 | 2.202 | 2.917 | 3.345 | 4.631 |

Assessment

| Sample: | 1 | 2 | 3 | 4 |
|---|---|---|---|---|
| Day 5 | 0.849 | 1.079 | 1.039 | 2.341 |
| Day 8 | 0.413 | 0.794 | 1.071 | 1.659 |

TABLE V

Mean Scaling Score

Preference

| Sample | 1 | 2 | 3 | 4 |
|---|---|---|---|---|
| Day 8 | 2.232 | 2.826 | 3.558 | 4.837 |

Assessment

| Sample: | 1 | 2 | 3 | 4 |
|---|---|---|---|---|
| Day 8 | 0.833 | 1.159 | 1.452 | 2.008 |

TABLE VI

Mean Fissuring Score

Preference

| Sample: | 1 | 2 | 3 | 4 |
|---|---|---|---|---|
| Day 8 | 2.321 | 3.083 | 3.500 | 4.619 |

Assessment

| Sample: | 1 | 2 | 3 | 4 |
|---|---|---|---|---|
| Day 8 | 0.532 | 0.770 | 0.928 | 1.682 |

On day 5 and 8, erythema assessment and preference scores for Sample 1 containing 10% sodium isethionate was significantly better than the control soap bar Samples 3 and 4. Sample 2 with 2.14% sodium isethionate was also found milder than either soap control Samples 3 and 4.

Scoring for scaling on Day 5, when the patch was freshly removed, was without statistical significance. The reason for insignificance was that the area was still moist due to occlusion. On Day 8, the scoring pattern was identical with the fissuring and erythema scoring pattern. Thus, the order of decreasing mildness was: Sample 1, 2, 3 and then 4. Sodium isethionate present at 10% inhibited scaling more than when present at 2%. Compositions lacking isethionate performed poorest in this area of skin protection.

Fissure scoring on Day 5 was statistically equivalent among all Samples. On Day 8, fissuring scores revealed skin damage with increasing severity in the order Sample 1, 2, 3 and then 4. The pattern for fissuring was, therefore, identical with that for erythema and scaling. These results all indicate that sodium

isethionate ameliorates skin damage caused by soap. Greater amounts of this skin mildness factor provide greater benefit.

The aforementioned Frosch-Kligman Test was conducted in the winter months. Score values for this evaluation were considerably higher as a group than that obtained with Samples of Example 1 taken during summer months.

Example 4

This Example illustrates the skin damage control effect of sodium isethionate as evaluated by the Flex Wash Procedure. Two toilet bars were prepared. Bar A was essentially composed of a 60/40 (tallow/coconut) sodium fatty acid soap base and served as the control sample. Bar B was identical to the control except that it contained 10% sodium isethionate, the ratio of sodium isethionate to soap being 0.11.

TABLE VII

Flex Wash Test of Sodium Isethionate

| Bar | Flex Wash Score |
|---|---|
| A (Control) | 30.1 |
| B | 26.2 |

In the experiment, 17 panellists, divided into two groups, participated in the study. Subjects of one group were asked to apply Bar B to their left arm and Bar A to their right arm. The other group did the opposite. Four daily readings for 5 consecutive days were obtained. The fourth reading each day was made 4 to 5 hours after the last treatment. This was when the peak of the erythema occurred. Erythema was judged according to a 7 point scale that ranged from 0 (no erythema) to 3 (deep erythema), *vide supra.* Treatment was discontinued on an arm if a reading of 2 or more was obtained on that arm. Treatment continued on the other arm.

A statistical analysis of the above experiment indicated that Bar B was significantly milder to the skin than the control without sodium isethionate. Statistical analysis gave an alpha level value of 0.0349 between the two soaps demonstrating the significance of the flex values. Further, control Bar A registrated a 2 on the erythema scale before the other arm, in 7 of the 17 panellists. There were no instances where Bar B registered a 2 before A. This is significant at the alpha level 0.0078, using the Sign Test.

Example 5

Sodium isethionate effects upon the skin were evaluated using the Guinea Pig Immersion Test. Two aqeuous solutions were prepared (control solution C contained 0.75% soap, 0.75% sodium acyl isethionate, 0.4% stearic acid and 0.07% sodium isethionate). Composition D was an aqueous solution identical to C except that the level of sodium isethionate was raised from 0.07% to 0.27%. The ratio of sodium isethionate to soap in solution C and D was 0.093 and 0.36, respectively. Immersion test results are outlined in the following Table.

Table VIII

Guinea Pig Immersion Test With Sodium Isethionate

| | Relative Response Scores | | |
|---|---|---|---|
| Composition | Erythema | Flaking | Roughness |
| C (Control) | 1.00 ± .19 | 0.68 ± 1.0 | 0.86 ± .20 |
| D | 0.67 | 0.40 | 0.47 |

Table VIII above demonstrates that as the level of sodium isethionate is raised relative response scores for all categories of skin damage (erythema, flaking, roughness) are lowered. The improvement was statistically significant.

Example 6

The present Example illustrates compositions wherein soap and sodium isethionate are combined with a second detergent active.

TABLE IX

Soap/Sodium Isethionate Formulations

| Component | Sample (% Weight) 1 | 2 | 3 | 4 | 5 | 6 | 7 |
|---|---|---|---|---|---|---|---|
| Sodium fatty acid soap | 50 | 50 | 80 | 70 | 70 | 70 | 70 |
| Sodium isethionate | 50 | 30 | 10 | 10 | 10 | 10 | 10 |
| Sodium acyl isethionate | | 20 | | | | | |
| Dodecyl benzene sulfonate | | | | 20 | | | |
| $C_6$-$C_{14}$ dialkyl sulphosuccinate ester | | | | | 20 | | |
| $C_{12}$-$C_{18}$ alkyl sulfate | | | | | | 20 | |
| $C_{12}$-$C_{18}$ alkyl ether sulfate | | | | | | | 20 |

Example 7

The following illustrates soap formulations containing various alkyl sodium isethionates.

TABLE X

Soap/Sodium Alkyl Isethionate Formulations

| Component | Sample (% Weight) 1 | 2 | 3 | 4 | 5 | 6 | 7 | 8 |
|---|---|---|---|---|---|---|---|---|
| Sodium fatty acid soap | 60 | 60 | 50 | 50 | 60 | 70 | 60 | 70 |
| Sodium 2-methyl isethionate | 40 | | 5 | | 10 | 2 | 20 | 10 |
| Sodium 2-hexyl isethionate | | 40 | | 5 | | | | |
| Sodium acyl isethionate | | | 45 | 45 | 30 | 28 | | |
| $C_6$-$C_{14}$ dialkyl sulfosuccinate ester | | | | | | | 20 | 20 |

**Claims**

1. A cleaning composition comprising:
(a) a fatty acid soap in an amount of from 30 wt% to 98 wt%;
(b) a mildness improving salt of following structure:

$$HO—CHRCH_2—SO_3\ M$$

where R is hydrogen or a $C_1$—$C_7$ alkyl or alkenyl radical; M is a cation selected from alkali metal, alkaline earth metal, ammonium, alkyl ammonium and mono-, di- or tri-alkanolammonium ions, in an amount of from 0.5 to 50 wt%; and wherein the ratio of soap (a) to mildness improving salt (b) ranges from 1:2 to 200:1; and
(c) $C_8$—$C_{18}$ free fatty acid in an amount of from 1 wt% to 30 wt%;
the composition containing no water soluble alkali metal fatty-acylaminomethane sulphonate wherein the fatty-acyl radical has from 8 to 18 carbon atoms.

2. Composition according to claim 1 further comprising one or more detergents other than soap.

3. Composition according to claim 2 comprising 5 wt% to 40 wt% of $C_8$—$C_{18}$ acyl isethionates.

4. Composition according to any one of the preceding claims wherein the mildness improving salt is sodium isethionate.

5. Composition according to any one of the preceding claims in liquid or in toilet bar form.

6. A composition according to claim 1 in toilet bar form comprising:
(a) from 55 wt% to 98 wt% alkali metal fatty acid soap;
(b) from 0.5 wt% to 30 wt% of sodium isethionate;
(c) from 5 wt% to 45 wt% sodium $C_8$—$C_{18}$ acyl isethionate; and
(d) from 1 wt% to 30 wt% free stearic acid.

**Patentansprüche**

1. Reinigungszusammensetzung, umfassend
(a) eine Fettsäure-Seife in einer Menge von 30 Gew.-% bis 98 Gew.-% und
(b) ein die Milde verbesserendes Salz der folgenden Struktur:

$$HO—CHRCH_2—SO_3\ M$$

worin R Wasserstoff oder ein $C_1$—$C_7$-Alkyl- oder Alkenylrest ist, M ein Kation, ausgewählt unter Alkalimetall, Erdalkalimetall, Ammonim, Alkylammonium und Mono-, Di- oder Trialkanolammoniumionen in einer Menge von 0,5 Gew.-% bis 50 Gew.-% ist, und worin das Verhältnis von Seife (a) zu die Mildeverbesserendes Salz (b) im Bereich von 1:2 bis 200:1 liegt, und
(c) $C_8$—$C_{18}$ freie Fettsäure in einer Menge von 1 Gew.-% bis 30 Gew.-%,
wobei die Zusammensetzung kein wasserlösliches Alkalimetall-Fettacylaminomethansulfonat enthält, worin der Fettacylrest 8 bis 18 Kohlenstoffatome aufweist.

2. Zusammensetzung nach Anspruch 1, weiterhin umfassend ein oder mehrere Detergentien, die nicht Seife sind.

3. Zusammensetzung nach Anspruch 2, umfassend 5 Gew.-% bis 40 Gew.-% $C_8$—$C_{18}$-Acylisethionate.

4. Zusammensetzung nach einem der vorhergehenden Ansprüche, worin das die Milde-verbessernde Salz Natriumisethionat ist.

5. Zusammensetzung nach einem der vorhergehenden Ansprüche in flüssiger oder in Toilettenstück-Form.

6. Zusammensetzung nach Anspruch 1, in Toilettenstück-Form, umfassend:
(a) 55 Gew-% bis 98 Gew.-% Alkalimetall-Fettsäure-Seife,
(b) 0,5 Gew.-% bis 30 Gew.-% Natriumisethionat,
(c) 5 Gew.-% bis 45 Gew.-% Natrium-$C_8$-$C_{18}$-Acylisethionat, und
(d) 1 Gew.-% bis 30 Gew.-% freie Stearinsäure.

**Revendications**

1. Composition de nettoyage comprenant:
(a) un savon d'acide gras en une proportion de 30 à 98% en poids;
(b) un sel d'amélioration de la douceur de formule:

$$HO—CHRCH_2—SO_3\ M$$

dans laquelle R est un atome d'hydrogène ou un radical alkyle ou alcényle en $C_{1-7}$; M est un cation choisi parmi les ions métal alcalin, métal alcalino-terreux, ammonium, alkylammonium et mono-, di- ou tri-

alcanolammonium à raison de 0,5 à 50% en poids; et dans laquelle le rapport du savon (a) au sel d'amélioration de la douceur (b) est compris entre 1:2; et

(c) un acide gras libre en $C_{8-18}$ en une proportion de 1 à 30% en poids,

la composition ne contenant pas d'aminométhane-sulfonate acylique gras de métal alcalin dans lequel le radical acyle gras contient de 8 à 18 atomes de carbone.

2. Composition selon la revendication 1, qui comprend en outre un ou plusieurs détergent(s) autre(s) que le savon.

3. Composition selon la revendication 2, comprenant de 5 à 40% en poids d'iséthionates acyliques en $C_{8-18}$.

4. Composition selon l'une quelconque des revendications précédentes, dans laquelle le sel d'amélioration de la douceur est l'iséthionate de sodium.

5. Composition selon l'une quelconque des revendications précédentes qui est sous forme d'un liquide ou d'un pain de toilette.

6. Composition selon la revendication 1 sous forme d'un pain de toilette comprenant:

(a) de 55 à 98% en poids d'un savon d'acide gras de métal alcalin;

(b) de 0,5 à 30% en poids d'iséthionate de sodium;

(c) de 5 à 45% en poids d'iséthionate acylique de sodium en $C_{8-18}$; et

(d) de 1 à 30% en poids d'acide stéarique libre.